# EUROPÄISCHE PATENTANMELDUNG

(11) **EP 3 330 697 A1**
(43) Veröffentlichungstag der Anmeldung: **06.06.2018**
(21) Anmeldenummer: 16201303.1
(22) Anmeldetag: 30.11.2016
(51) Int. Cl.: G01N 21/25

(54) **VORRICHTUNG ZUR ERMITTLUNG DER WIRKUNG VON WIRKSTOFFEN AN NEMATODEN UND ANDEREN ORGANISMEN IN WÄSSRIGEN TESTS**

(71) Anmelder: Bayer Aktiengesellschaft, 51373 Leverkusen (DE)
(72) Erfinder: Fois, Franco, 40789 Monheim (DE); Harnau, Michael, 42799 Leichlingen (DE); Ochmann, Klaus, 51377 Leverkusen (DE)
(74) Vertreter: BIP Patents

(57) **Zusammenfassung**

Die Erfindung betrifft eine Vorrichtung (1) zur Ermittlung der Wirkung von Wirkstoffen an Nematoden und anderen Organismen in wässrigen Tests, umfassend eine Halterung (40) zur Aufnahme einer Zellkulturplatte (50) mit Kavitäten (57), in die sich die Nematoden mit den Wirkstoffen füllen lassen, wobei die Zellkulturplatte (50) eine Unterseite (51), eine Oberseite (62) sowie mehrere Seitenflächen aufweist, die sich zwischen Unterseite (51) und Oberseite (52) erstrecken, eine Kamera (20), die dazu dient, Bilder von vorzugsweise der Unterseite (51) der Zellkulturplatte (50) aufzunehmen, und eine Beleuchtungseinrichtung (30) zur Ausleuchtung der Zellkulturplatte (50), wobei die Beleuchtungseinrichtung (30) mehrere Lichtquellen umfasst. Erfindungsgemäß ist vorgesehen, dass in Einsatzlage der Zellkulturplatte (50) das Licht einer ersten Lichtquelle (31) durch eine erste Seitenfläche (53), das Licht einer zweiten Lichtquelle (32) durch eine zweite Seitenfläche (54), das Licht einer dritten Lichtquelle (33) durch eine dritte Seitenfläche (55), und das Licht einer vierten Lichtquelle (34) durch eine vierte Seitenfläche (56) von außen in die Zellkulturplatte (50) tritt. Zudem betrifft die Erfindung ein Verfahren zur Justierung der einzelnen Lichtquellen.

## Beschreibung

Die Erfindung betrifft eine Vorrichtung zur Ermittlung der Wirkung von Wirkstoffen an Nematoden und anderen Organismen in wässrigen Tests. Des Weiteren betrifft des Weiteren ein Verfahren zur Justierung einer Beleuchtungseinrichtung der Vorrichtung.

Viele Arten von Nematoden (Fadenwürmer) stellen Schädlinge in der Landwirtschaft dar, da sie durch ihr Eindringen in die Wurzelsysteme den Pflanzenstoffwechsel stark beeinträchtigen können. Gegen einen Nematodenbefall sind bereits verschiedene chemische Substanzen, die sogenannten Nematiziden, entwickelt worden. Jedoch besteht ein großer Bedarf, weitere Wirkstoffe zu identifizieren, durch die Nematoden wirkungsvoll bekämpft werden können.

Aus der WO 2016/116291 A1 ist eine Vorrichtung zur Ermittlung der Wirkung von Wirkstoffen an Nematoden bekannt, mit der eine Vielzahl von Wirkstoffen in kurzer Zeit getestet werden können. Dabei werden gleichzeitig die einzelnen mit Nematoden und unterschiedlichen Wirkstoffen befüllten Kavitäten einer Zellkulturplatte untersucht. Die durch eine Halterung der Vorrichtung gehaltene Zellkulturplatte weist dabei eine Unterseite, eine Oberseite sowie vier Seitenflächen auf, die sich zwischen der Unterseite und der Oberseite der Zellkulturplatte erstrecken. Die Vorrichtung umfasst eine Kamera, die dazu dient, Bilder von der Unterseite der Zellkulturplatte und damit allen Kavitäten gleichzeitig aufzunehmen. Eine Beleuchtungseinrichtung der Vorrichtung weist zwei sich gegenüberstehende Lichtquellen auf, die die Zellkulturplatte ausleuchten.

Es hat sich gezeigt, dass die Anwendung der Vorrichtung der WO 2016/116291 A1 bei der Untersuchung von sehr kleinen Fadenwürmen wie Dirofilarien ihre Grenzen findet. Die Auflösung der Vorrichtung lässt sich zwar grundsätzlich durch Auswahl einer entsprechend höher auflösenden Kamera steigern, doch hat sich herausgestellt, dass Ungleichmäßigkeiten in der Ausleuchtung der Zellkulturplatte bei größerer Auflösung softwaretechnisch nicht mehr in befriedigendem Maße korrigiert werden können. Eine zuverlässige und belastbare Ermittlung der Wirkung der Wirkstoffe ist somit bei sehr kleinen Organismen nicht mehr möglich.

Der Erfindung liegt daher die Aufgabe zu Grunde, eine Vorrichtung zur Ermittlung der Wirkung von Wirkstoffen an Nematoden und anderen Organismen bereitzustellen, durch die auch bei sehr kleinen Organismen wie Dirofilarien eine zuverlässige Ermittlung der Wirkung der Wirkstoffe möglich ist.

Die der Erfindung zu Grunde liegende Aufgabe wird durch die Merkmalskombination nach Anspruch 1 gelöst. Ausführungsbeispiele der Erfindung können den Unteransprüchen zu Anspruch 1 entnommen werden.

Die erfindungsgemäße Vorrichtung zeichnet sich dadurch aus, dass das Licht einer ersten Lichtquelle durch eine erste Seitenfläche, dass Licht einer zweiten Lichtquelle durch eine zweite Seitenfläche, das Licht einer dritten Lichtquelle durch eine dritte Seitenfläche und das Licht einer vierten Lichtquelle durch eine vierte Seitenfläche jeweils von außen in die Zellkulturplatte treten. Somit tritt von allen Seitenflächen Licht in das Innere der Zellkulturplatte ein. Durch diese Maßnahme ist grundsätzlich eine gleichmäßige Ausleuchtung der Zellkulturplatte im Sinne der Erfindung möglich. Bei entsprechend leistungsfähiger Kamera kann somit die Wirkung von Wirkstoffen auch auf sehr kleine Organismen untersucht werden. Somit können belastbare Ergebnisse bei einer Auflösung von 30 µm und kleiner erreicht werden. In einem Ausführungsbeispiel beträgt die Auflösung weniger als 20 µm, beispielsweise 15 µm.

Für die erste Lichtquelle kann eine Intensität des Lichts individuell eingestellt werden. Dies gilt sinngemäß auch für die zweite Lichtquelle, die dritte Lichtquelle und die vierte Lichtquelle. Beispielsweise kann somit die Intensität des Lichts der ersten Lichtquelle unabhängig von der Intensität des Lichts der zweiten Lichtquelle oder einer anderen Lichtquelle eingestellt werden. Letztlich können also die vier Lichtquellen mit jeweils unterschiedlichen Lichtintensitäten Licht in die Zellkulturplatte emittieren.

Die erste Lichtquelle kann seitlich neben der ersten Seitenfläche angeordnet sein, sodass in Einsatzlage der Zellkulturplatte (die Zellkulturplatte erstreckt sich dabei in einer horizontalen Ebene) ein Lichtstrahl der ersten Lichtquelle im Wesentlichen horizontal durch die erste Seitenfläche in die Zellkulturplatte gelangt. Etwaige Umlenkspiegel oder andere optische Umlenkungen können somit entfallen.

Die Ausführungen zu der ersten Lichtquelle sollen auch sinngemäß für die anderen Lichtquellen gelten. So kann beispielsweise auch die zweite Lichtquelle seitlich neben der zweiten Seitenfläche angeordnet sein. Auch die im Folgenden beschriebenen Merkmale, die sich auf die erste Lichtquelle beziehen, können bei einer Einzelnen der übrigen Lichtquellen, bei einigen der übrigen Lichtquellen oder auch bei allen übrigen Lichtquellen verwirklicht sein.

Die Erfindung geht von einer rechteckigen Zellkulturplatte aus, die eine viereckige Grundform mit jeweils zwei parallelen Seitenflächen aufweist. Beispielsweise kann das Verhältnis von langen Kanten (langen Seitenflächen) zu kurzen Kanten (kurze Seitenflächen) 3 zu 2 betragen. Die Kavitäten sind üblicherweise in mehreren, zueinander parallel angeordneten Reihen angeordnet. Beispielsweise kann eine Zellkulturplatte 8 Reihen mit jeweils 12 Kavitäten aufweisen.

Der Erfindung liegt die Erkenntnis zu Grunde, dass bei der Untersuchung von sehr kleinen Organismen die erforderliche gute Ausleuchtung mit einer Beleuchtungseinrichtung möglich ist, wenn Licht von allen Seitenflächen in die Zellkulturplatte tritt. Bei einer sechseckigen Zellkulturplatte, deren entsprechende Grundform aber derzeit unüblich ist, müssten gemäß der Erfindung alle sechs Seitenflächen mit Licht bestrahlt werden. Bei einer dreieckigen Zellkulturplatte wäre die Verwendung von nur drei Lichtquellen ausreichend.

Die erste Seitenfläche, die sich zwischen Oberseite und Unterseite der Zellkulturplatte erstreckt, kann durch eine lichtdurchlässige Wand geschlossen sein. Sie kann aber auch (vollständig) offen sein. Dies gilt auch für alle übrigen Seitenflächen.

Die erste Lichtquelle kann sich über die gesamte erste Seitenfläche erstrecken, wodurch eine gleichmäßige Bestrahlung der ersten Seitenfläche möglich ist.

In einem Ausführungsbeispiel lassen sich für die erste Lichtquelle eine vertikale Höhe bezogen auf die Zellkulturplatte, ein Neigungswinkel um eine eigene Längsachse und/oder ein horizontaler Abstand zu der ersten Seitenfläche individuell einstellen.

Durch die flexible und individuell einstellbare Einstellung einer jeden Lichtquelle kann die Ausleuchtung der Zellkulturplatte optimiert werden. Jede Lichtquelle ist dabei räumlich gesehen dreidimensional verstellbar (Höhe, horizontaler Abstand, Neigungswinkel). Zusätzlich kann eine vierte Dimension eingestellt werden, nämlich die Lichtintensität. Es hat sich überraschend herausgestellt, dass auch bei einer symmetrischen Zellkulturplatte mit rechteckiger Grundform eine individuelle Einstellung der Lichtquelle an jeder der Seitenflächen für die Qualität der Messungen von großer Bedeutung ist.

Eine vertikale Höhe des aus der ersten Lichtquelle austretenden Lichtstrahls kann 2 bis 6 mm, vorzugsweise 3 bis 5 mm betragen. Bei seitlicher Anordnung der ersten Lichtquelle tritt ohne Neigungswinkel dieser Lichtstrahl horizontal durch die erste Seitenfläche, also zwischen Oberseite und Unterseite in die Zellkulturplatte ein.

Vorzugsweise ist ein Öffnungswinkel des Lichtstrahls sehr klein, damit beispielsweise eine Deckfolie, mit der die einzelnen Kavitäten der Zellkulturplatte abgedeckt sind, nicht ausgeleuchtet wird und somit keine Störeffekte hervorrufen kann. Jedoch können auch größere Werte für den Öffnungswinkel toleriert werden, wenn beispielsweise durch die Kamera nur ein kleiner vertikaler Abschnitt der Zellkulturplatte scharf abgebildet wird, so dass die Ebene der Zellkulturplatte, in der sich die Deckfolie befindet, nicht mehr scharf ist.

Die erste Lichtquelle kann eine Reihe von nebeneinander angeordneten Leuchtdioden (LED) aufweisen. Um über eine wirksame Länge der ersten Lichtquelle ein gleichmäßiges Licht zu erzeugen, kann die erste Lichtquelle eine Fresnel-Linsenanordnung aufweisen. Durch diese Fresnel-Linsenanordnung emittiert somit die erste Lichtquelle einen Lichtstrahl, dessen Intensität über die Längserstreckung der Lichtquelle gesehen konstant ist.

Die Halterung für die Zellkulturplatte kann mehrere Aufnahmeecken aufweisen, wobei eine erste Aufnahmeecke zur Aufnahme eines Endes der ersten Seitenfläche und eine zweite Aufnahmeecke zur Aufnahme eines gegenüberliegenden Endes der ersten Seitenfläche dienen. Ein Abstand zwischen der ersten Aufnahmeecke und der zweiten Aufnahmeecke ist dabei vorzugsweise größer als eine Länge einer Reihe von Kavitäten, die sich entlang der ersten Seitenfläche erstreckt. Das Licht der ersten Lichtquelle, kann somit über die gesamte Länge der Reihe von Kavitäten ungehindert in die Zellkulturplatte eintreten. Vorzugsweise ist die Halterung so ausgebildet, dass zwischen der ersten Lichtquelle und der ersten Seitenfläche der Zellkulturplatte keine sonstigen Hindernisse angeordnet sind, um einen ungehinderten und nicht verschatteten Eintritt des Lichts der ersten Lichtquelle in die Zellkulturplatte zu ermöglichen.

Die Kamera kann ein telezentrisches Objektiv umfassen, um einen perspektivischen Fehler des Bildes der Zellkulturplatte an deren Ränder auszuschließen oder zumindest weitestgehend auszuschließen. Durch das telezentrische Objektiv kann zudem eine geringe Tiefenschärfe eingestellt werden, wodurch Störeffekte, beispielsweise verursacht durch die oben erwähnte Deckfolie für die Kavitäten, reduziert erreicht werden können.

Im Betrieb der Vorrichtung nimmt die Kamera von der gesamten Unterseite beispielsweise einer 96 Kavitäten umfassenden Zellkulturplatte in zeitlichen Abständen von beispielsweise 1 bis 5 Sekunden mehrere digitale Bilder auf. Durch eine Auswertung dieser Bilder anhand einer Pixelanalyse wird für jede einzelne Kavität eine Kennzahl für die mittlere Geschwindigkeit ermittelt, mit der sich die in dieser Kavität befindlichen Dirofilarien bewegen. In einer Kavität mit einem wirksamen Wirkstoff ist die mittlere Geschwindigkeit dabei grundsätzlich kleiner als in einer Kavität ohne Wirkstoff. Auf Basis der mittleren Geschwindigkeit lässt sich somit die Wirksamkeit eines Wirkstoffes abschätzen. In diesem Zusammenhang wird auf die WO 2016/116291 A1 verwiesen, in der detailliert ein entsprechendes Verfahren zur Ermittlung der Wirkung von Wirkstoffen beschrieben wird, mit welchen die erfindungsgemäße Vorrichtung betrieben werden kann.

Bei einer ganz gleichmäßig ausgeleuchteten Zellkulturplatte und bei jeweils gleich gefüllten Kavitäten (beispielsweise jeweils ohne Wirkstoff) müsste sich theoretisch für jede Kavität eine gleiche mittlere Geschwindigkeit für die Bewegung der Dirofilarien ergeben, wenn die zwangsläufig vorhandene biologische Streuung außer Acht gelassen wird. Bei zu schlechter Ausleuchtung der Kavitäten weichen hingegen die Ergebnisse trotz gleicher Befüllung sehr deutlich voneinander ab. Bei einer derart ausgeleuchteten Zellkulturplatte mit unterschiedlichen Wirkstoffen befüllten Kavitäten wäre daher eine Aussage über die einzelnen Wirkstoffe nicht belastbar. Durch die Beleuchtungseinrichtung der erfindungsgemäßen Vorrichtung ist es jedoch möglich, den Einfluss der in der Praxis nicht ganz auszuschließenden Unterschiede in der Ausleuchtung so gering zu halten, dass eine belastbare Aussage über die Wirkung von Wirkstoffen auch bei sehr kleinen Dirofilarien (also bei entsprechend hoher Auflösung) möglich ist.

Eine weitere Aufgabe, die Bereitstellung eines Verfahrens zur Justierung der Beleuchtungseinrichtung der oben in ihren verschiedenen Ausführungen beschriebenen Vorrichtung, wird durch die Merkmalskombination gemäß Anspruch 10 gelöst. Ausführungsbeispiele des erfindungsgemäßen Verfahrens können den Unteransprüchen zu Anspruch 10 entnommen werden.

Das erfindungsgemäße Verfahren sieht vor, dass für jede der Lichtquellen die relative Höhe bezogen auf die Zellkulturplatte, der Neigungswinkel, der horizontale Abstand zur angrenzenden Seitenfläche sowie die Lichtintensität individuell eingestellt werden, um eine möglichst gleichmäßige Ausleuchtung der einzelnen Kavitäten der Zellkulturplatte zu erhalten.

In einem systematischen Prozess können iterativ einzelne Parameter (Höhe, Abstand, Neigungswinkel, Lichtintensität) für jede Lichtquelle eingestellt werden und mit diesen eingestellten Parametern jeweils eine oben beschriebene Ermittlung der mittleren Geschwindigkeit für jede gleich befüllte Kavität durchgeführt werden. Dieser Vorgang kann beliebig oft wiederholt werden. Je weniger die ermittelten Ergebnisse für die mittlere Geschwindigkeit dabei voneinander abweichen, desto geringer ist der Einfluss, den die nicht gleichmäßige Ausleuchtung der Zellkulturplatte auf die Versuchsergebnisse hat. Weichen die Ergebnisse schließlich nur noch in einer Größenordnung voneinander ab, die auf die biologische Streuung zurück zu führen ist, kann das Verfahren der Justierung beendet werden.

Das Verfahren zur Justierung der Beleuchtungseinrichtung kann vorsehen, dass mit der Kamera ein Bild eines einfarbigen Blatts erstellt wird, dass sich in einer Ebene oberhalb einer Zellkulturplatte befindet (beispielsweise kann auf eine in der Halterung befindlichen nicht-gefüllten Zellkulturplatte unter Zwischenschaltung einer weiteren leeren Zellkulturplatte das Blatt angeordnet werden) und dass anhand einer Auswertung des Bildes die Justierung erfolgt bzw. verfeinert wird. Beispielsweise kann von dem Bild ein Histogramm der Lichtintensität erzeugt werden und auf Basis dieses Histogramms die Justierung erfolgen. Eine gleichmäßige Ausleuchtung des Blatts ist dann gegeben, wenn das dazugehörige Histogramm für alle Unterbereiche (dies können auch die einzelnen Pixel des digitalen Bildes sein) jeweils eine gleiche Lichtintensität ausweist, was bedeuten würde, dass das Histogramm aus lediglich einem Balken mit dieser einen Lichtintensität besteht.

In einem Ausführungsbeispiel wird die Beleuchtungseinrichtung justiert einerseits mittels der Ergebnisse einer Zellkulturplatte mit gleich befüllten Kavitäten und zusätzlich mittels des oben beschriebenen Blatts. Die (Erst-)Justierung anhand der gleich befüllten Zellkulturplatte und der (Nach-)Justierung durch das Blatt kann mehrmals hintereinander und/oder in umgekehrter Reihenfolge erfolgen.

Zudem kann am Ende einer Justierung die Lichtintensität der einzelnen Lichtquellen um jeweils einen gleichen Betrag oder proportional reduziert werden, so dass sich für die mittlere Lichtintensität des Bilds des einfarbigen Blatts Papier ein vorgegebener Wert (beispielsweise ein Mittelwert von 128, wenn die Lichtintensität Werte von 0 bis 256 annehmen kann). Im Betrieb der erfindungsgemäßen Vorrichtung wird dann jeder Kavität der Zellkulturplatte ein entsprechender Bereich auf dem Blatt Papier zugeordnet. Die durch die Kamera ermittelten Werte der Lichtintensität für die Pixel der Kavität können dann anhand der Lichtintensität des entsprechenden Bereichs auf dem Blatt Papier normiert werden. Ist beispielsweise für eine Kavität ein eher hellerer Bereich des Blatt Papiers ermittelt worden, werden die für diese Kavität ermittelten Werte für die Lichtintensität durch die Normierung reduziert.

Anhand eines in den Figuren dargestellten Ausführungsbeispiels soll die Erfindung näher erläutert werden. Es zeigen:
- Figur 1: schematisch den Aufbau einer erfindungsgemäßen Vorrichtung;
- Figur 2: schematisch eine Zellkulturplatte und eine Beleuchtungsvorrichtung mit vier Lichtquellen; und
- Figur 3: einen Schnitt entlang der Linie III-III in Figur 2.

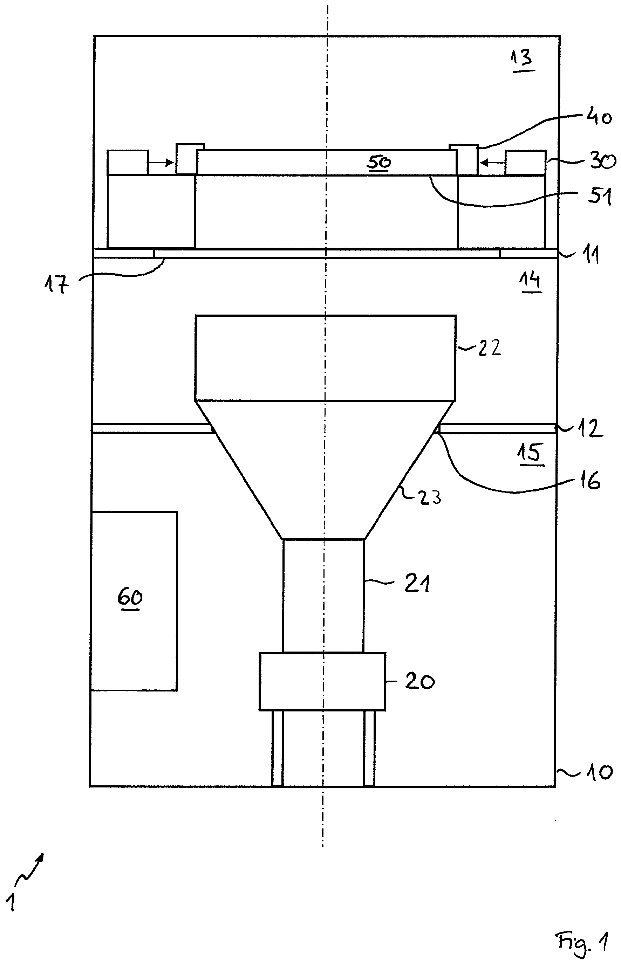

Figur 1 zeigt schematisch eine Vorrichtung zur Ermittlung der Wirkung von Wirkstoffen an Dirofilarien und anderen Organismen in wässrigen Tests. Die Vorrichtung umfasst, welche in ihrer Gesamtheit mit 1 bezeichnet wird, ein Gehäuse 10 mit einer oberen Trennplatte 11 und einer mittleren Trennplatte 12. Die Trennplatten 11, 12 unterteilen einen von dem Gehäuse 10 umgebenen Innenraum in einen oberen Bereich 13, einen mittleren Bereich 14 und einen unteren Bereich 15. In dem unteren Bereich 15 ist eine Kamera 20 montiert. Die Kamera 20 weist ein telezentrisches Objektiv 21 auf, dessen von der Kamera 20 abgewandtes Ende 22 in den mittleren Bereich 14 des Gehäuses 10 ragt. Die mittlere Trennplatte 12 weist eine kreisrunde Öffnung 16 auf, durch die das Objektiv 21 hindurchgreift. Ein kegelstumpfförmiger Abschnitt 23 des Objektivs 21 stützt sich dabei an dem kreisrunden Rand der Öffnung 16 ab.

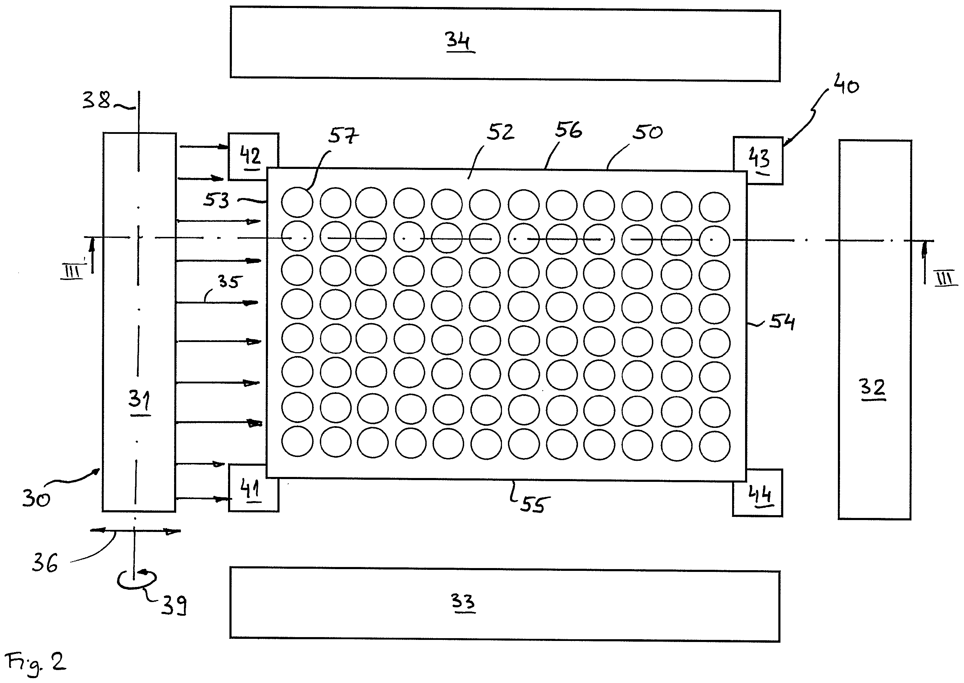

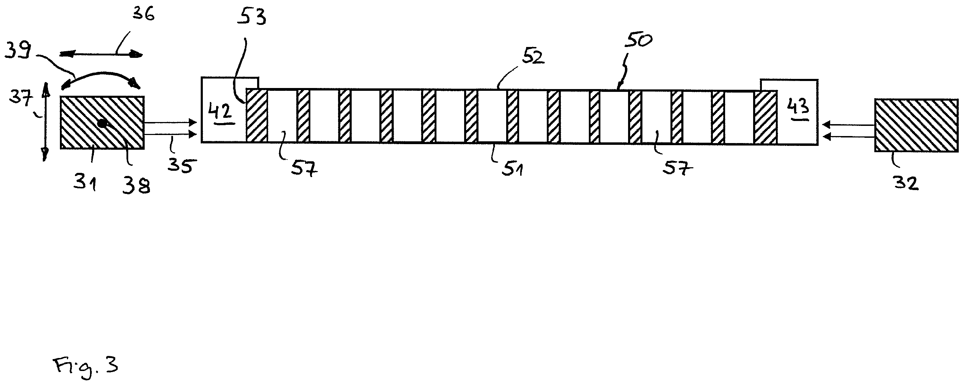

In dem oberen Bereich 13 des Gehäuses 10 ist eine Beleuchtungseinrichtung 30 sowie eine Halterung 40 für eine Zellkulturplatte 50 untergebracht. Die Beleuchtungseinrichtung 30, die Halterung 40 und die Zellkulturplatte 50 werden anhand der Figuren 2 und 3 im Folgenden noch näher beschrieben.

Neben der Kamera 20 nimmt der untere Bereich 15 des Gehäuses 10 auch eine Steuerung 60 für die Kamera 20 und die Beleuchtungseinrichtung 30 auf. Die Steuerung 60 kann dabei aus separaten Steuereinheiten für die Kamera 20 und die Beleuchtungseinrichtung 30 bestehen. Die Steuerung 60 lässt sich an einen Computer anschließen.

Mit der erfindungsgemäßen Vorrichtung 1 können von einer Unterseite 51 der Zellkulturplatte 50 erzeugt werden. In der Trennplatte 11 ist daher eine Glasscheibe 17 eingefasst.

Figur 2 zeigt von oben die Beleuchtungseinrichtung 30, die eine erste Lichtquelle 31, eine zweite Lichtquelle 32, eine dritte Lichtquelle 33 und eine vierte Lichtquelle 34 aufweist. Die erste Lichtquelle 31 umfasst mehrere Leuchtdioden (nicht dargestellt), die in einer Reihe nebeneinander angeordnet sind und einen über nahezu die gesamte Länge der ersten Lichtquelle 31 einen streifenförmigen Lichtstrahl 35 emittieren. Der Lichtstrahl ist durch die parallelen Pfeile 35 dargestellt. Auch die übrigen Lichtquellen 32, 33, 34 emittieren jeweils einen streifenförmigen Lichtstrahl über ihre nahezu vollständige Länge in Richtung der Zellkulturplatte 50. Der Übersicht halber sind aber entsprechende Pfeile zur Kennzeichnung der jeweiligen Lichtstrahlen in Figur 2 nicht dargestellt.

Die Zellkulturplatte 50 weist neben der oben bereits erwähnten Unterseite 51 eine Oberseite 52 sowie eine erste Seitenfläche 53, eine zweite Seitenfläche 54, eine dritte Seitenfläche 55 und eine vierte Seitenfläche 56 auf. Aufgrund der rechteckigen Grundform der Zellkulturplatte 50 verlaufen die sich gegenüberstehenden Seitenflächen 53, 54 senkrecht zu dem anderen Paar von parallel verlaufenden Seitenflächen 55, 56.

Die Zellkulturplatte 50 weist eine Vielzahl von Kavitäten 57 auf, die in 8 Reihenmit jeweils 12 Kavitäten angeordnet sind. Wie der Figur 3 entnommen werden kann, weist jede einzelne Kavität 57 im Längsschnitt eine U-förmige Form auf. Jede Kavität 57 lässt sich dabei von oben, also von der Oberseite 52 her mit einer wässrigen Lösung befüllen, in der sich die Dirofilarien und ein Wirkstoff befinden. Nach erfolgter Befüllung können die Kavitäten über eine Deckfolie abgedeckt werden.

Wie in der Zusammenschau der Figuren 2 und 3 deutlich wird, geben die einzelnen Lichtquellen 31, 32, 33, 34 jeweils Licht durch die Seitenflächen 53, 54, 55, 56 in die Zellkulturplatte 50 ab. So tritt Licht aus der ersten Lichtquelle von außen durch die erste Seitenfläche 53 in die Zellkulturplatte 50. Das gleiche gilt sinngemäß für die Lichtquellen 32, 33, 34. Um eine möglichst gute Ausleuchtung der einzelnen Kavitäten 57 der Zellkulturplatte 50 zu erreichen, lässt sich die Position der Lichtquelle 31 in Bezug auf die Zellkulturplatte 50 verändern.

Durch den Doppelpfeil 36 ist angedeutet, dass sich ein Abstand zwischen der ersten Lichtquelle 31 und der ersten Seitenfläche 53 der Zellkulturplatte 50 vergrößern oder verkleinern lässt. Der Doppelpfeil 37 (siehe Figur 3) zeigt an, dass sich die erste Lichtquelle 31 bezogen auf die Zellkulturplatte 50 nach oben oder nach unten bewegen lässt. Zudem kann die erste Lichtquelle 31 um eine Längsachse 38, die parallel zur ersten Seitenfläche 53 der Zellkulturplatte 50 verläuft, gekippt werden. Die entsprechende Verstellbarkeit um die Längsachse 38 oder um eine Achse, die parallel dazu verläuft, ist durch den gebogenen Pfeil 39 gekennzeichnet. Zudem lässt sich über die Steuerung 60 die Lichtintensität der ersten Lichtquelle 31 einstellen. Der horizontale Abstand zu der ersten Seitenfläche, die vertikale Lage, der Neigungswinkel und die Lichtintensität der ersten Lichtquelle können von den entsprechenden Einstellparametern der übrigen Lichtquellen 32, 33, 34 individuell eingestellt werden. Somit ist es beispielsweise möglich, die Beleuchtungseinrichtung derart zu justieren, dass die Lichtintensität der ersten Lichtquelle 31 nicht nur von den Lichtintensitäten der dazu quer angeordneten Lichtquellen 33, 34 abweicht, sondern auch von der Lichtintensität der gegenüberliegenden Lichtquelle 32.

Die Halterung 40 weist eine erste Aufnahmeecke 41, eine zweite Aufnahmeecke 42, eine dritte Aufnahmeecke 43 und eine vierte Aufnahmeecke 44 auf. Vorzugsweise sind dabei drei der vier Aufnahmeecken 41, 42, 43, 44 als Festanschläge ausgebildet, während die verbleibende vierte Aufnahmeecke als gefederter Anschlag ausgebildet ist. Ein Abstand zwischen beispielsweise der ersten Aufnahmeecke und der zweiten Aufnahmeecke ist so bemessen, dass der Lichtstrahl 35 der ersten Lichtquelle 31 ungehindert auf den Bereich der ersten Seitenfläche treten kann, in dem sich die Kavitäten 57 befinden. Mit anderen Worten ist ein Abstand zwischen den Aufnahmeecken 41, 42 größer als eine Länge der ersten Spalte der Kavitäten, die sich entlang der ersten Seitenfläche 53 erstreckt.

Aufgrund der unterschiedlichen Längen der längeren Seitenflächen 55, 66 und der kürzeren Seitenflächen 53, 54 sind auch die Leuchtquellen 33, 34 einerseits und die Lichtquellen 31, 32 andererseits unterschiedlich lang. Die Breite eines Lichtstrahls aus einer Lichtquelle ist dabei immer länger als die Länge des Bereichs der zugehörigen Seitenfläche, in dem sich die Kavitäten befinden.

Durch die Beleuchtungseinrichtung 30 und die besondere Anordnung der einzelnen Aufnahmeecken der Halterung 40 werden alle Bereiche der Seitenflächen der Zellkulturplatte 50, in denen sich die Kavitäten 57 befinden, mit Licht aus den Lichtquellen beaufschlagt. Durch die individuelle Einstellbarkeit der einzelnen Lichtquellen lässt sich die Beleuchtungseinrichtung 30 so justieren, dass eine annähernd optimale Ausleuchtung der Kavitäten 57 erreicht wird. Ein bevorzugtes Kontrollkriterium für die möglichst optimale Ausleuchtung ist oben bereits beschrieben. Von einer optimalen Ausleuchtung ist dann auszugehen, wenn bei einer Zellkulturplatte, deren einzelne Kavitäten mit jeweils dem gleichen Wirkstoff befüllt worden sind, die kavitätsspezifischen Ergebnisse hinsichtlich der Wirksamkeit des jeweils gleichen Wirkstoffes nicht voneinander abweichen bzw. eine Standardabweichung aufweisen, die in etwa der geschätzten Standardabweichung aufgrund der biologischer Organismen entspricht.

### Bezugszeichenliste

- 1: Vorrichtung

- 10: Gehäuse
- 11: obere Trennplatte
- 12: mittlere Trennplatte
- 13: oberer Bereich
- 14: mittlerer Bereich
- 15: unterer Bereich
- 16: Öffnung
- 17: Glasplatte

- 20: Kamera
- 21: Objektiv
- 22: Ende
- 23: kegelstumpfförmiger Bereich

- 30: Beleuchtungseinrichtung
- 31: erste Lichtquelle
- 32: zweite Lichtquelle
- 33: dritte Lichtquelle
- 34: vierte Lichtquelle
- 35: Pfeil (Lichtstrahl)
- 36: Doppelpfeil
- 37: Doppelpfeil
- 38: Pfeil

- 40: Halterung
- 41: erste Aufnahmeecke
- 42: zweite Aufnahmeecke
- 43: dritte Aufnahmeecke
- 44: vierte Aufnahmeecke

- 50: Zellkulturplatte
- 51: Unterseite
- 52: Oberseite
- 53: erste Seitenfläche
- 54: zweite Seitenfläche
- 55: dritte Seitenfläche
- 56: vierte Seitenfläche
- 57: Kavität

- 60: Steuerung

## Patentansprüche

1. Vorrichtung (1) zur Ermittlung der Wirkung von Wirkstoffen an Nematoden und anderen Organismen in wässrigen Tests, umfassend
- eine Halterung (40) zur Aufnahme einer Zellkulturplatte (50) mit Kavitäten (57), in die sich die Nematoden mit den Wirkstoffen füllen lassen, wobei die Zellkulturplatte (50) eine Unterseite (51), eine Oberseite (62) sowie mehrere Seitenflächen aufweist, die sich zwischen Unterseite (51) und Oberseite (52) erstrecken,
- eine Kamera (20), die dazu dient, Bilder von vorzugsweise der Unterseite (51) der Zellkulturplatte (50) aufzunehmen,
- eine Beleuchtungseinrichtung (30) zur Ausleuchtung der Zellkulturplatte (50),
wobei die Beleuchtungseinrichtung (30) mehrere Lichtquellen umfasst, **dadurch gekennzeichnet, dass** in Einsatzlage der Zellkulturplatte (50)
- das Licht einer ersten Lichtquelle (31) durch eine erste Seitenfläche (53),
- das Licht einer zweiten Lichtquelle (32) durch eine zweite Seitenfläche (54),
- das Licht einer dritten Lichtquelle (33) durch eine dritte Seitenfläche (55), und
- das Licht einer vierten Lichtquelle (34) durch eine vierte Seitenfläche (56)
von außen in die Zellkulturplatte (50) tritt.

2. Vorrichtung (1) nach Anspruch 1, **dadurch gekennzeichnet, dass** für die erste Lichtquelle (31) eine Intensität des Lichts individuell einstellbar ist.

3. Vorrichtung (1) nach Anspruch 1 oder 2, **dadurch gekennzeichnet, dass** die erste Lichtquelle (31) seitlich neben der ersten Seitenfläche (53) angeordnet ist.

4. Vorrichtung (1) nach einem der Ansprüche 1 bis 3, **dadurch gekennzeichnet, dass** sich die erste Lichtquelle (31) über die gesamte Länge der ersten Seitenfläche (63) erstreckt.

5. Vorrichtung (1) nach einem der Ansprüche 1 bis 4, **dadurch gekennzeichnet, dass** für die erste Lichtquelle (31) eine vertikale Höhe bezogen auf die Zellkulturplatte, ein horizontaler Abstand zu der ersten Seitenfläche und ein Neigungswinkel um eine Längsachse (38) der ersten Lichtquelle (31) individuell einstellbar ist.

6. Vorrichtung (1) nach einem der Ansprüche 1 bis 5, **dadurch gekennzeichnet, dass** eine vertikale Höhe eines aus der ersten Lichtquelle (31) austretenden Lichtstrahls 2 bis 6 mm beträgt.

7. Vorrichtung (1) nach einem der Ansprüche 1 bis 6, **dadurch gekennzeichnet, dass** die erste Lichtquelle (31) eine Fresnel-Linsenanordnung aufweist, um über die wirksame Länge der ersten Lichtquelle (31) ein gleichmäßiges Licht zu erzeugen.

8. Vorrichtung (1) nach einem der Ansprüche 1 bis 7, **dadurch gekennzeichnet, dass** die Halterung (40) mehrere Aufnahmeecken aufweist, wobei eine erste Aufnahmeecke (41) zur Aufnahme eines Endes der ersten Seitenfläche (53) und eine zweiten Aufnahmeecke (42) zur Aufnahme eines gegenüberliegenden Endes der ersten Seitenfläche (53) dient, und wobei ein Abstand zwischen der ersten Aufnahmeecke (41) und der zweiten Aufnahmeecke (42) größer ist als eine Länge einer Reihe oder Spalte von Kavitäten (57), die sich entlang der ersten Seitenfläche (53) erstreckt.

9. Vorrichtung (1) nach einem der Ansprüche 1 bis 8, **dadurch gekennzeichnet, dass** die Kamera (20) eine telezentrisches Objektiv (21) umfasst.

10. Verfahren zur Justierung einer Beleuchtungseinrichtung (30) einer Vorrichtung (1) nach einem der Ansprüche 1 bis 9, **dadurch gekennzeichnet, dass** bezogen auf die Zellkulturplatte die relative Höhe einer jeden Lichtquelle, der Neigungswinkel einer jeden Lichtquelle sowie eine Lichtintensität einer jeden Lichtquelle individuell eingestellt werden, um eine gute Ausleuchtung der einzelnen Kavitäten (57) der Zellkulturplatte (50) zu erhalten.

11. Verfahren nach Anspruch 10, **dadurch gekennzeichnet, dass** mit der Kamera (20) ein Bild eines einfarbigen Blatts erstellt wird, das sich in einer Ebene oberhalb der ausgeleuchteten Zellkulturplatte (50) befindet und dass anhand einer Auswertung des Bilds die Justierung erfolgt.

12. Verfahren nach Anspruch 10, **dadurch gekennzeichnet, dass** von dem Bild ein Histogramm der Lichtintensität erzeugt wird und auf Basis dieses Histogramms die Justierung erfolgt.
